# EUROPEAN PATENT SPECIFICATION

(11) **EP 2 145 649 B1**
(45) Date of publication and mention of the grant of the patent: **11.04.2012**
(21) Application number: 08425484.6
(22) Date of filing: 17.07.2008
(51) Int. Cl.: A61N 5/06, A61H 15/02

(54) **Device for application of phototherapy**
Vorrichtung zur Anwendung von Phototherapie
Dispositif pour l'application de photothérapie

(43) Date of publication of application: 20.01.2010
(73) Proprietor: ERREVI, Di Pelis Enrica, 24061 Albano S. Alessandro (BG) (IT)
(72) Inventor: Rocchi, Agostino, 24066 Pedrengo (Bergamo) (IT)
(74) Representative: Lunati, Vittoriano

(56) References cited:
- EP-A- 1 847 759
- WO-A-98/20937
- WO-A-03/001984
- WO-A1-2007/040691
- JP-A- 2007 233 085
- US-A- 5 259 380
- US-A1- 2002 188 334

## Description

The present invention concerns a device for the application of phototherapy of the type described in the preamble of the first claim.

Medical and beauty treatments which use light energy, also called phototherapy, are currently known. For example by patent documents: WO-A-98/20937; WO-A-2007/040691; US-B-5259380; US 2002/0 188 334.

In particular treatments performed with laser devices and treatments performed with LED (light emitting diode) devices are known.

Of these, the treatments that use laser light are based on the heat and thermal lesion of the light on the treated portion of the human body.

Said laser light treatments therefore exploit the high power per surface unit which lasers are able to provide.

However, laser treatments have an important secondary effect.

The high temperature produced on the skin surface by the lasers can lead to destruction of the skin cells.

In order to avoid said secondary effect, careful medical administration of phototherapy with laser light and application of light impulses for very short periods are necessary.

Phototherapy performed with LED devices, on the other hand, is not ablative, i.e. it does not damage the tissues and does not have significant side effects or contraindications.

In fact, phototherapy applied with LED devices has a photochemical and not a photothermal effect. It stimulates the photoreceptors which, in turn, stimulate different cellular surface processes.

Said phototherapy with LED devices is used in particular for the regeneration of tissue.

Besides the advantages cited, however, phototherapy with LED devices also has drawbacks.

In fact, the results of phototherapy applied by means of LED devices are inferior to phototherapy applied with laser devices and it does not always provide the desired results.

LED devices, in fact, do not permit use of a high power per surface unit. Furthermore, LED phototherapy only permits the treatment of blemishes and diseases in surface tissues and does not allow said problems to be treated at a greater depth.

In this situation the technical aim of the present invention is to conceive a device for the application of phototherapy able to substantially remedy the drawbacks cited.

In the context of said technical aim, an important object of the invention is to produce a device for the application of phototherapy able to guarantee optimal cell regeneration of the tissues treated.

A further important object of the invention is to obtain a device for the application of phototherapy which does not excessively heat the tissues treated or have similar secondary effects.

A further object of the invention is to conceive a device for the application of phototherapy comprising LED devices which permit the application of high powers.

The technical aim and the objects specified are achieved by a device for the application of phototherapy as described in the attached claim 1.

The accompanying drawings illustrate exemplary embodiments. In particular:

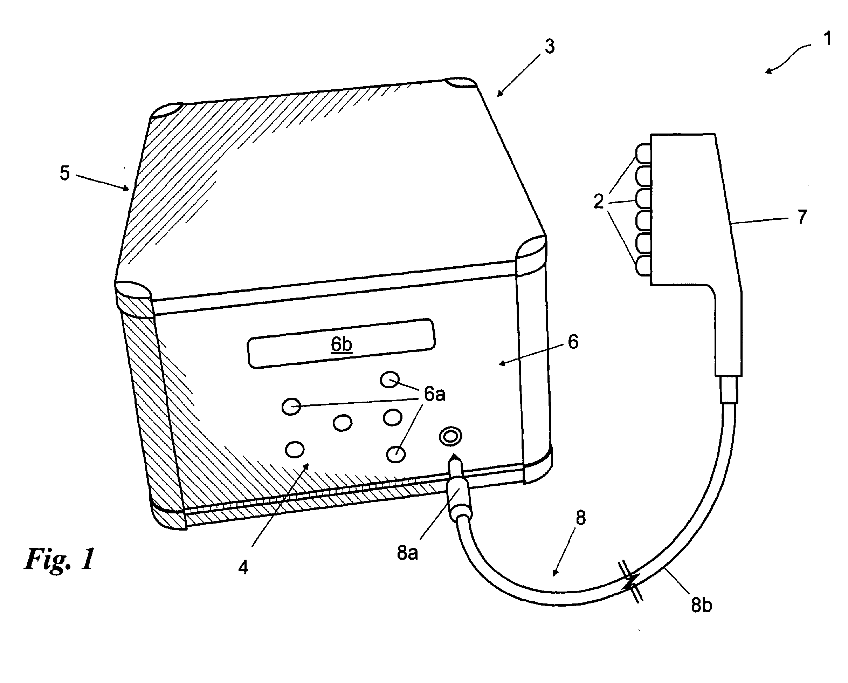

**Fig. 1** shows an overview of the device
**Fig. 2a** illustrates a first detail of the device;
**Fig. 2b** highlights a second detail of the device;
**Fig. 2c** shows a third detail of the device; and
**Fig. 3** is a graph illustrating the switch-on and switch-off alternation of the light for the phototherapy treatment.

With reference to the Figures, the device according to the invention is indicated overall by the number **1**.

It is suitable for applying phototherapy treatments on human and animal patients and in particular treatments for cellular regeneration of the cutaneous and subcutaneous tissues.

It comprises at least one LED **2**, and preferably several LEDs 2, suitable for emitting electromagnetic waves for phototherapy treatments, suitable for emitting electromagnetic waves near the frequencies of the visible spectrum, means for control **3** of the device 1 suitable for activating the LEDs 2, i.e. activating the emission for certain time intervals of the electromagnetic waves of the LEDs 2, and second control means 4 of the device 1 suitable for allowing the user to adjust and activate the first control means 3.

Activation of the LEDs 2 does not correspond to their being continuously switched on, i.e. to a continuous emission of electromagnetic waves, but to an alternation of regular cycles **20** for switching the same on and off.

In particular the LEDs 2 are activated at constant switch-on and switch-off frequencies and for constant duty cycles. The term duty cycle identifies the ratio between the time interval **21** in which the LEDs 2 are active and the interval of time 22 in which the LEDs 2 are off; the term period 20 indicates the sum of the time interval 21 in which the leds are active added to the time interval 22 in which they are off.

The LEDs 2 are activated at switch-on and switch-off frequencies between 0.1 Hz and 1 kHz and with duty cycle below 2%.

Furthermore the control means 3 are suitable for activating the LEDs 2 at a plurality of different frequencies and a plurality of different duty cycles, and the second control means 4 allow the user at least to select the activation frequencies of the LEDs 2.

The second control means 4 allow the user to choose the activation periods **23**. The term activation period 23 indicates a period during which the LEDs 2 are activated at one single and constant switch-on and switch-off frequency. Said activation periods 23 have durations in the order of minutes and involve the running of thousands of switch-on and switch-off cycles 20 of the LEDs 2. Furthermore the second control means 4 allow the user to set entire treatments **24**, consisting of one or more periods of activation 23.

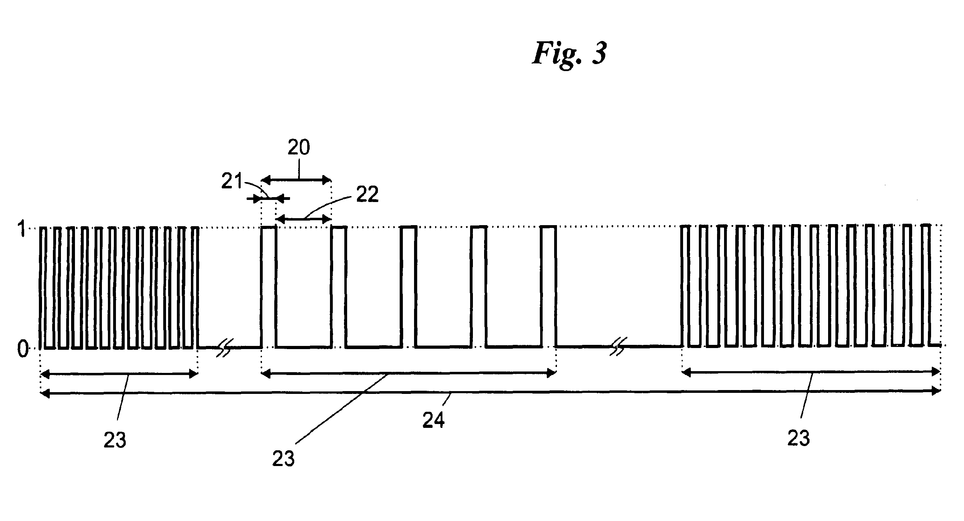

Fig. 3 schematises the alternation of switch-on and switch-off of the LEDs 2, according to the time for an entire treatment 24. In particular in said graph the value on the Y axis indicates switch-on corresponding to 1, or switch-off corresponding to 0, of the LEDs 2. In the graph the duration of the switch-on and switch-off cycles 20, which determines the switch-on and switch-off frequency, has been considerably increased for graphic representation purposes.

Furthermore the control means 3 activate the LEDs 2 at pre-selected switch-on and switch-off frequencies. Each pre-selected frequency is associated with a constant pre-selected value of the duty cycle and a constant pre-selected value of the activation power of the LEDs 2. More specifically, several programmes can be selected, each with a relative pre-selected duty cycle, power and frequency.

The activation periods 23, alternation thereof and the duration of the treatment 24 have a time duration which can be set by the user and are not pre-selected.

The LEDs 2 are suitable for emitting electromagnetic waves with wavelength between 800 nm and 1100 nm, and in particular with wavelength near to 880 nm.

As is known, the LEDs 2 consist of at least one chip made of a semiconductor material and two electrical connections, one positive and one negative, suitable for transmitting the electric current to the chip. The chips stimulated by the electric current emit electromagnetic waves at specific frequencies according to the semiconductor material of which the chip is made. Lastly, the LEDs comprise a transparent bulb suitable for covering the chip and appropriately directing the electromagnetic waves.

Each LED 2 comprises one single bulb and a plurality of chips. Several chips are scheduled for each LED 2.

Due to this solution each LED 2 emits a peak power higher than 900 mW. Lastly, the LEDs 2 have a bulb made of transparent biocompatible polymeric material and with a diameter of between 0.5 cm and 2 cm and a height between 0.5 cm and 2 cm.

Structurally the device 1 comprises an external casing **5** which includes the control means 3 and a control panel **6**, comprising a push-button panel **6a** and a screen **6b**.

The control panel 6 substantially constitutes the second control means 4.

The control means 3, on the other hand, consist of a computer, known per sé and suitable for transferring electric current to the LEDs 2, in the selected switch-on intervals 21.

The device 1 furthermore comprises at least one mobile unit 7 including the LEDs 2 and suitable for being manually handled.

The unit 7 furthermore comprises a connection **8**, consisting of a plug **8a** and an electrical cable **8b**, which can be inserted in the casing 5.

It furthermore comprises a handle **9** and a supporting element **10** for the LEDs 2.

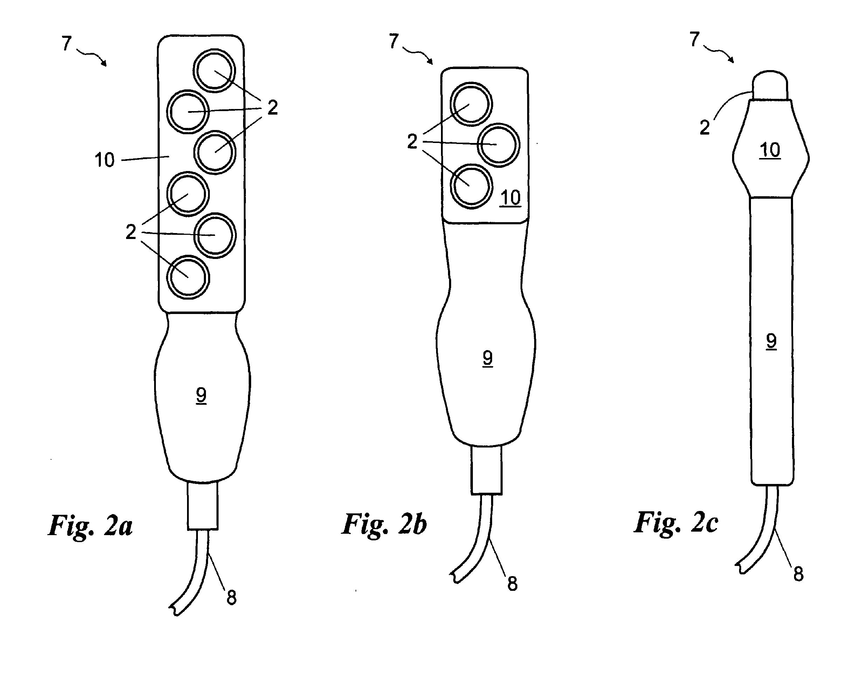

In particular three units 7 are present, which can be selectively connected to the casing 5, comprising six, three and one single LED 2, as illustrated in Fig. 2a-2c.

The supporting element 10 of the units 7 including three and six LEDs 2 comprises a flat surface on which the LEDs 2 are positioned. In particular on the unit 7 comprising six LEDs 2, said LEDs 2 are arranged in two rows of three LEDs 2 side by side and offset, as illustrated in Fig. 2a. In the same way on the unit 7 comprising three LEDs 2, said LEDs 2 are arranged not aligned, as illustrated in Fig. 2b.

The operation of a device 1, described above in structural terms, is the following.

The device 1 is connected to the mains or similar and used by a user to administer phototherapy treatment to patients.

The casing 5 is then connected to a unit 7, chosen according to the area of the body to be treated. In particular, the unit 7 with six LEDs 2 is used for the abdomen or legs, unit 7 comprising three LEDs 2 is used for the arms, while the unit 7 with one single LED 2 is used for the face.

The user then selects, via the control means 3, a specific programme, and automatically the relative duty cycle and the relative emission power, of the switch-on/switch-off cycles 20 of the LEDs 2.

The user selects, again via the second control means 4 and in particular the panel 6, the duration of the period of activation 23 with the programme previously selected.

The control means 3 then activate the control means 4, and in particular the computer, which store the selected settings and send to the LEDs 2, via the described connections 8, electrical impulses with the programme selected, thus triggering the described switch-on intervals 21 of the LEDs 2 and substantially administering to the patient the phototherapy treatment 24.

During the treatment 24 the user massages with the unit 7, with the surface formed by the LEDs 2, the part of the body treated, thus administering not only a phototherapeutic but also a massotherapeutic treatment.

The invention offers important advantages.

The device 1 suitable for applying the treatments 24 with the switch-on and switch-off frequencies described, allows high powers to be used without resulting in a significant and harmful increase in the temperature of the cells treated. Said result is due in particular to the high switch-on/switch-off frequencies described and furthermore to the reduced duty cycles applied.

Furthermore the device 1 permits customisation of the treatment 24 according to the patient, within appropriately pre-selected frequencies, thus maximising the results.

The higher performances are applicable in particular due to appropriate choice of the LEDs 2 comprising a plurality of chips and therefore suitable for withstanding high powers.

Furthermore the device 1 comprises LEDs 2 which emit electromagnetic radiations at a wavelength suitable for penetrating inside the tissues and therefore for treating not only the surface tissues.

In particular it has been ascertained that the electromagnetic radiations of the wavelengths described have a penetration inside the human body estimated to be in the order of 3.5 cm.

A further advantage is the described form and dimension of the LEDs 2 which allow for mechanical massage of the human body in addition to phototherapy. The devices 1 described have resulted, in laboratory experiments, in an average increase of 25% in regeneration of the surface tissue cells.

Modifications and variations can be made to the invention falling within the scope of the inventive concept.

All the details can be replaced by equivalent elements and any materials, forms and dimensions can be used.

## Claims

1. Device for the application of phototherapy comprising:
- at least one LED (2) suitable for emitting electromagnetic waves with wavelength between 800 nm and 1100 nm,
- first control means (3) suitable for activating said at least one LED (2),
- second control means (4) of said device (1) suitable for allowing a user to adjust and activate said first control means (3),
- at least one unit (7) including said at least one LED (2)
wherein said device is further adapted to be manually handled during the treatment for massage of a part of a body with the surface formed by said at least on LED (2),
**characterized by**
- said first control means (3) being suitable for activating said at least one LED (2) with switch-on and switch-off frequency between 0.1Hz and 1 kHz and for duty cycles below 2%,
- and said at least one LED (2) comprising a bulb and a plurality of chips.

2. Device as claimed in claim 1, wherein said first control means (3) are suitable for activating said LEDs (2) with a plurality of switch-on and switch-off frequencies and a plurality of duty cycles.

3. Device as claimed in claim 2, wherein said control means allow the user to select a plurality of said switch-on and switch-off frequencies (23) for a plurality of activation periods (24) and to select the duration of said activation periods (24).

4. Device as claimed in claim 2 or 3, wherein said switch-on and switch-off frequencies are pre-selected and wherein each frequency is combined with one single duty cycle and one single switch-on power.

5. Device as claimed in one or more of the preceding claims, wherein each of said LEDs (2) comprises six chips.

6. Device as claimed in claim 5, wherein each of said LEDs (2) is suitable for withstanding a peak power of at least 900 mW.

7. Device as claimed in one or more of the preceding claims, wherein said LEDs (2) emit electromagnetic waves with wavelength of 880 nm.

8. Device as claimed in claim 1, wherein one of said units (7) comprises six LEDs (2).

9. Device as claimed in claim 8, wherein said LEDs (2) have a diameter of between 0.5 cm and 2 cm and a height of between 0.5 cm and 2 cm and are positioned on said handle on the same flat surface (10a) and in two rows, each one consisting of three LEDs (2), side by side.

10. Device as claimed in claim 1, wherein one of said units (7) comprises three LEDs (2).

11. Device as claimed in claim 10, wherein said LEDs (2) have a diameter of between 0.5 cm and 2 cm and a height of between 0.5 cm and 2 cm and are arranged on said unit (7) on the same flat surface (10a).

12. Device as claimed in claim 1, wherein one of said units (7) comprises one single LED (2).

## Patentansprüche

1. Vorrichtung zur Anwendung von Phototherapie, umfassend:
- mindestens eine LED (2), geeignet zum Aussenden elektromagnetischer Wellen mit einer Wellenlänge zwischen 800 nm und 1100 nm;
- erste Kontrollmittel (3), geeignet zur Aktivierung der besagten mindestens einen LED (2);
- zweite Kontrollmittel (4) der besagten Vorrichtung (1), geeignet, einem Benutzer zu erlauben, die besagten ersten Kontrollmittel (3) zu regulieren und zu aktivieren;
- mindestens eine Einheit (7), die die besagte mindestens eine LED (2) umfasst, wobei die besagte Vorrichtung weiterhin geeignet ist, während der Massagebehandlung eines Körperteils mit der Oberfläche, die aus der besagten mindestens einen LED (2) gebildet wird, manuell gehandhabt zu werden, **gekennzeichnet dadurch**,
- das die besagten ersten Kontrollmittel (3) geeignet sind, die besagte mindestens eine LED (2) mit einer Einschalt- und Ausschaltfrequenz zwischen 0,1 Hz und 1 kHz und für Arbeitszyklen unter 2% zu aktivieren;
- und dass die besagte eine LED (2) eine Lampe und eine Vielzahl von Chips umfasst.

2. Vorrichtung gemäß Anspruch 1, bei der die besagten ersten Kontrollmittel (3) geeignet sind, die besagten LEDs (2) mit einer Vielzahl von Einschalt- und Ausschaltfrequenzen und einer Vielzahl von Arbeitszyklen zu aktivieren.

3. Vorrichtung gemäß Anspruch 2, bei der die besagten Kontrollmittel dem Benutzer erlauben, eine Vielzahl der besagten Einschalt- und Ausschaltfrequenzen (23) für eine Vielzahl von Aktivierungszeiten (24) zu wählen und die Dauer der besagten Aktivierungszeiten (24) zu wählen.

4. Vorrichtung gemäß Anspruch 2 oder 3, bei der die besagten Einschalt- und Ausschaltfrequenzen vorher ausgewählt werden und bei der jede Frequenz mit einem einzigen Arbeitszyklus und einer einzigen Einschaltleistung kombiniert sind.

5. Vorrichtung gemäß einem oder mehreren der vorangegangenen Ansprüche, bei der jede der besagten LEDs (2) sechs Chips umfasst.

6. Vorrichtung gemäß Anspruch 5, bei der jede der besagten LEDs (2) geeignet ist, eine Spitzenleistung von mindestens 900 mW auszuhalten.

7. Vorrichtung gemäß einem oder mehreren der vorangegangenen Ansprüche, bei der die besagten LEDs (2) elektromagnetische Wellen mit einer Wellenlänge von 880 nm aussenden.

8. Vorrichtung gemäß Anspruch 1, bei der eine der besagten Einheiten (7) sechs LEDs (2) umfasst.

9. Vorrichtung gemäß Anspruch 8, bei der die besagten LEDs (2) einen Durchmesser zwischen 0,5 cm und 2 cm und eine Höhe zwischen 0,5 cm und 2 cm besitzen und auf dem besagten Griff auf der gleichen ebenen Oberfläche (10a) in zwei Reihen, von denen jede aus drei LEDs (2) besteht, Seite an Seite positioniert sind.

10. Vorrichtung gemäß Anspruch 1, bei der eine der besagten Einheiten (7) drei LEDs (2) umfasst.

11. Vorrichtung gemäß Anspruch 10, bei der die besagten LEDs (2) einen Durchmesser zwischen 0,5 cm und 2 cm und eine Höhe zwischen 0,5 cm und 2 cm besitzen und auf der besagten Einheit (7) auf der gleichen ebenen Oberfläche (10a) angeordnet sind.

12. Vorrichtung gemäß Anspruch 1, bei der eine der besagten Einheiten (7) eine einzige LED (2) umfasst.

## Revendications

1. Dispositif pour l'application de photothérapie, comprenant:
- au moins une diode électroluminescente (abrégé LED) (2) apte à émettre des ondes électromagnétiques dont la longueur d'onde est entre 800 nm et 1100 n,
- des premiers moyens de commande (3) destinés à activer ladite au moins une diode électroluminescente (2),
- des deuxièmes moyens de commande (4) dudit dispositif (1) aptes à permettre qu'un utilisateur puisse régler et activer lesdits premiers moyens de commande (3),
- au moins une unité (7) comprenant ladite au moins une diode électroluminescente (2),
dans lequel ledit dispositif est en outre apte à être manié manuellement pendant le traitement pour le massage d'une partie d'un corps avec la surface formée par ladite au moins une diode électroluminescente (2),
**caractérisé par**
- lesdits premiers moyens de commande (3) qui sont aptes à activer ladite au moins une diode électroluminescente (2) à une fréquence d'allumage et d'extinction entre 0,1 Hz et 1 kHz et pendant des cycles d'utilisation au-dessous de 2%,
- et ladite au moins une diode électroluminescente (2) qui comprend une ampoule et une pluralité de puces.

2. Dispositif selon la revendication 1, dans lequel lesdits premiers moyens de commande (3) sont aptes à activer lesdites LED (2) selon une pluralités de fréquences d'allumage et d'extinction et une pluralité de cycles d'utilisation.

3. Dispositif selon la revendication 2, dans lequel lesdits moyens de commande permettent que l'utilisateur sélectionne une pluralité desdites fréquences d'allumage et d'extinction (23) pour une pluralité de périodes d'activation (24) et sélectionne la durée desdites périodes d'activation (24).

4. Dispositif selon la revendication 2 ou 3, dans lequel lesdites fréquences d'allumage et d'extinction sont pré-sélectionnées et dans lequel chaque fréquence se combine à un seul cycle d'utilisation et une seule puissance d'allumage.

5. Dispositif selon une ou plusieurs des revendications précédentes, dans lequel chacune desdites LED (2) comprend six puces.

6. Dispositif selon la revendication 5, dans lequel chacune desdites LED (2) est apte à résister à une puissance de pointe d'au moins 900 mW.

7. Dispositif selon une ou plusieurs des revendications précédentes, dans lequel lesdites LED (2) émettent des ondes électromagnétiques ayant une longueur d'onde de 880 nm.

8. Dispositif selon la revendication 1, dans lequel une desdites unités (7) comprend six LED (2).

9. Dispositif selon la revendication 8, dans lequel lesdites LED (2) ont un diamètre compris entre 0,5 cm et 2 cm et une hauteur comprise entre 0,5 cm et 2 cm et sont positionnées sur ladite poignée sur la même surface plate (10a) et le long de deux rangées, chacune comportant trois LED côte à côte.

10. Dispositif selon la revendication 1, dans lequel une desdites unités (7) comprend trois LED (2).

11. Dispositif selon la revendication 10, dans lequel lesdites LED (2) ont un diamètre compris entre 0,5 cm et 2 cm et une hauteur comprise entre 0,5 cm et 2 cm est sont disposées sur ladite unité (7) sur la même surface plate (10a).

12. Dispositif selon la revendication 1, dans lequel une desdites unités (7) comprend une seule LED (2).
